# EUROPÄISCHE PATENTSCHRIFT

(11) **EP 0 278 454 B1**
(45) Veröffentlichungstag und Bekanntmachung des Hinweises auf die Patenterteilung: **27.01.1993**
(21) Anmeldenummer: 88101793.3
(22) Anmeldetag: 08.02.1988
(51) Int. Cl.: C07D 213/81, A61K 31/44

(54) **Pyridin-2,4- und 2,5-dicarbonsäure-Derivate, Verfahren zu ihrer Herstellung, Verwendung derselben sowie Arzneimittel auf Basis dieser Verbindungen**
2,4- and 2,5-Pyridine-dicarboxylic-acid derivatives, process for their preparation, their use and medicines based on these compounds
Dérivés d'acide pyridine-2,4- et 2,5-dicarboxyliques, leur procédé de préparation, leur utilisation et médicaments à base de ces composés

(30) Priorität: 10.02.1987 DE 3703962
(43) Veröffentlichungstag der Anmeldung: 17.08.1988
(73) Patentinhaber: HOECHST AKTIENGESELLSCHAFT, 65926 Frankfurt am Main (DE)
(72) Erfinder: Brocks, Dietrich, Dr., D-6200 Wiesbaden 42 (DE); Burghard, Harald, Dr., D-6384 Schmitten 3 (DE); Günzler, Volkmar, Dr., D-3550 Marburg-Cappel (DE); Hanauske-Abel, Hartmut, Dr., D-6501 Dexheim (DE)

(56) Entgegenhaltungen:
- DE-A- 3 432 094
- JOURNAL OF THE AMERICAN CHEMICAL SOCIETY, Band 107, Nr. 13, 26. Juni 1985, Seite 3A; A.G. TALMA et al.: "Reductions of activated carbonyl compounds with chiral bridged 1,4-dihydropyridines. An investigation of scope and structural effects"
- JOURNAL OF MEDICINAL CHEMISTRY, Band 10, Nr. 4, July 1967, Seiten 3A-4A; G.J. ATWELL et al.: "Potential antitumor agents. V. Bisquaternary salts"

## Beschreibung

Verbindung, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie α,αʹ-Dipyridyl zu einer Hemmung der C1_{q}-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4- und -2,5-dicarbonsäure effektiv gehemmt wird (K. Mayama et al., Eur. J. Biochem. 138 (1984) 239-245). Diese Verbindungen sind in der Zellkultur allerdings nur in sehr hohen Konzentrationen als Hemmstoffe wirksam (V. Günsler et al. Collagen and Rel. Research 3, 71 1983).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Überraschenderweise wurde nun gefunden, daß die α-Aminosäure-, α-Aminosäureester-, Di- oder Tripeptid-Derivate der Pyridin-2,4- und -2,5-dicarbonsäure ausgezeichnete Hemmstoffe der Kollagenbiosynthese im Tiermodell sind.

Der eigentliche Wirkstoff, die Pyridin-2,4- oder 2,5-Dicarbonsäure entsteht erst durch Hydrolyse der α-Aminosäure-, α-Aminosäureester-, Di- oder Tripeptid-Derivate in der Zelle. Die α-Aminosäure, α-Aminosäureester-, Di- oder Tripeptid-Derivate können auf Grund ihrer höheren Lipophilie und der Tatsache, daß sie während des Transports überaschenderweise nur sehr langsam hydrolysiert werden bis in die Zellen transportiert werden. Erst hier wird der eigentliche Wirkstoffe, Pyridin-2,4 oder -2,5-dicarbonsäure, freigesetzt.

Die Erfindung betrifft somit:
1. Pyridin-2,4- oder -2,5-dicarbonsäure-Derivate der Formel I, 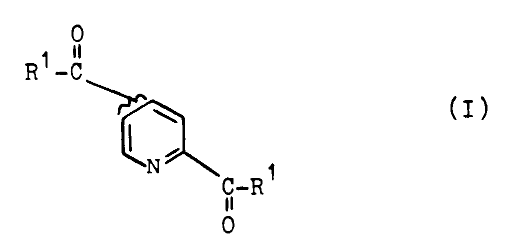 worin
   - R¹: über N-Terminus gebundene α-Aminosäure oder α-Aminosäurealkylester oder α-Aminosäureamid oder α-Aminosäurealkyl- oder -dialkylamid,
   wobei die genannten Alkylreste 1 bis 4 C-Atome aufweisen und gegebenenfalls mit Phenyl monosubstituiert sind und wobei die C₃- und C₄-Alkylreste auch verzweigt sein können
   oder
   - R¹: über N-Terminus gebundenes Di- oder Tripeptid
   bedeutet
   sowie deren physiologisch verträgliche Salze.
2. Bevorzugt sind Pyridin-2,4- oder -2,5-dicarbonsäure-Derivate der Formel I
   worin
   - R¹: über N-Terminus gebundene α-Aminosäure oder α-Aminosäurealkylester,
   wobei der Alkylrest 1 bis 3 C-Atome aufweist und gegebenenfalls mit Phenyl monosubstituiert ist und wobei der C₃-Alkylrest auch verzweigt sein kann
   bedeutet
   sowie deren physiologisch verträgliche Salze.

Weiterhin betrifft die Erfindung ein Verfahren zur Herstellung von Pyridin-2,4- oder -2,5-dicarbonsäure-Derivaten der Formel I, das dadurch gekennzeichnet ist, daß man eine Verbindung der Formel II, 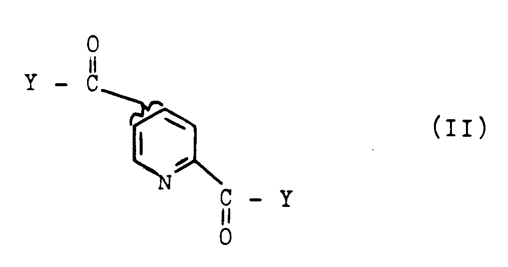
mit einer Verbindung der Formel III

R¹ - H (III)

umsetzt,
wobei R¹ die zu Formel I angegebenen Bedeutungen hat und Y Halogen oder Hydroxy ist oder zusammen mit der Carbonylgruppe einen Aktivester oder ein Anhydrid bildet
und wobei für den Fall, daß R¹ ein über N-Terminus gebundenes Di- oder Tripeptid oder eine über N-Terminus gebundene α-Aminosäure ist, die vorhandene(n) freie(n) Carboxyfunktion(en) gegebenenfalls geschützt ist (sind) und wobei man diese gegebenenfalls verhandene(n) Schutzgruppe(n) nach der Umsetzung hydrolytisch oder hydrogenolytisch unter Bildung der freien Carboxylfunktion(en) abspaltet
und die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen - sofern sie nicht käuflich sind - näher beschrieben.

Als temporäre Carboxylschutzgruppen sind Esterschutzgruppen geeignet, wie sie auch in der Peptidsynthese Anwendung finden (vergleiche z.B. Kontakte Merck 3/79, Seiten 15 und 19 ff).
Häufig verwendet werden die Methyl-, Benzyl- oder tert.-Butylester, ferner ONbzl, OMbzl, OPic. Die Abspaltung erfolgt je nach Schutzgruppe durch saure oder alkalische Hydrolyse oder durch Hydrierung in Gegenwart eines Übergangsmetall-Katalysators (Houben-Weyl, Methoden der organischen Chemie, Band E5, Seiten 496-504, vierte Auflage, 1985).

Die Herstellung der erfindungsgemäßen Verbindungen gelingt am einfachsten dadurch, daß die beiden Komponenten, das Pyridin-Derivat gemäß Formel (II) und die α-Aminosäure bzw. das α-Aminosäurederivat gemäß Formel (III), in äquimolaren Mengen oder bis zu einem etwa 5-fachen Überschuß an III, vermengt werden und bei Temperaturen zwischen -30 bis 150°C, bevorzugt bei 20 bis 100 °C bis zur Beendigung der Reaktion umgesetzt werden. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel, wie Diäthyläther oder Dimethoxyäthan oder Tetrahydrofuran, chlorierten Kohlenwasserstoffen wie Methylenchlorid, Chloroform, Tri- oder Tetrachloräthylen, Benzol, Toluol oder auch polaren Lösungsmitteln wie Dimethylformamid oder Aceton oder Dimethylsulfoxid arbeitet. Auch hier kann ein Überschuß von α-Aminosäure bzw. α-Aminosäurederivat gemäß Formel (III), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130°C besonders bevorzugt sind.

Gegebenenfalls kann die Umsetzung auch in Gegenwart von Basen erfolgen. Als zusätzliche Basen kommen anorganische Säurefänger wie Carbonate oder Hydrogencarbonate z.B. Natrium- oder Kaliumcarbonat oder Natrium- oder Kaliumhydrogencarbonat, oder organische Säurefänger wie tertiäre Amine, wie Triäthylamin, Tributylamin, Äthyldiisopropylamin oder heterocyclische Amine wie N-Alkylmorpholin, Pyridin, Chinolin oder Dialkylaniline in Betracht.

Bevorzugt erfolgt die Umsetzung der Verbindungen gemäß Formel (II) mit den α-Aminosäuren bzw. α-Aminosäurederivaten gemäß Formel (III) unter Zusatz eines wasserabspaltenden Mittels wie Dialkylcarbodiimid, wobei die Alkylreste 1 bis 8 C-Atome aufweisen, die im Falle der C₃-C₈-Verbindungen auch verzweigt oder cyclisch sein können; bevorzugt wird Dicyclohexylcarbodiimid eingesetzt. Eine entsprechende Methode ist in Houben-Weyl, Bd. XV/2, Seiten 103-111, Methoden der Organischen Chemie, 4. Aufl., Georg Thieme Verlag, Stuttgart, 1974, beschrieben.

Gegebenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion, Bernstein-Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (II) erhält man beispielsweise durch Umsetzung von Pyridin-2,4- oder -2,5-dicarbonsäure (II, Y = Hydroxy) zu dem entsprechenden Pyridin-2,4- oder -2,5-dicarbonsäurehalogenid, bevorzugt -chlorid (II, Y = Halogen) (nach literaturbekannten Verfahren, z.B. Organikum, Organisch Chemisches Grundpraktikum, 15. Aufl., VEB Deutscher Verlag der Wissenschaften, 1976, S. 595 ff), welches dann mit einem geeigneten Alkohol, z.B. Paranitrobenzylalkohol zu dem entsprechenden Aktivester (II, Y = Aktivester) umgesetzt wird. Ebenso kann die Pyridin-2,4- oder -2,5-dicarbonsäure auch zunächst unter Zusatz einer geeigneten Carbonsäure oder Carbonsäureester wie Chlorameisensäureethylester in ein gemischtes Anhydrid (II, Y = Anhydrid) überführt werden, welches dann mit den α-Aminosäuren oder α-Aminosäure-Derivaten zu den erfindungsgemäßen Produkten umgesetzt wird. Eine entsprechende Methode ist z.B. in Houben-Weyl, Methoden der organischen Chemie, Band XV/2, Seiten 169-183, vierte Auflage, 1974, Georg Thieme Verlag Stuttgart, beschrieben.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ III oder der N-bzw. C-terminalen Quervernetzungsdomäne des Kollagens Typ IV (7s-Kollagen bzw. Typ-IV-Kollagen-NC₁) im Serum bestimmt werden.

Zu diesem Zweck wurden die Hydroxyprolin-, Prokollagen-III-Peptid-, 7s-Kollagen- und Typ-IV-Kollagen-NC₁-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, S. 335-476, New York, Academic Press, 1964).

Die pharmakologische Wirksamkeit der erfindungsgemäßen Substanzen wurde untersucht; dabei zeigte sich eine deutliche Hemmung der Prolin- und Lysinhydroxylase.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositorien oder Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konzervierungs-, Stabilisierungs-, Netz-oder Emulgiermittel, Salze zur Veränderung des osmotischen Drucks oder Puffer. Sie können auch noch andere therapeutisch wirksame Stoffe enthalten.

Im folgenden wird die Erfindung anhand von Beispielen näher erläutert:

### Beispiele

1. Pyridin-2,4-dicarbonsäure-bis(1-methoxycarbonylethyl)amid
   1.02 g Pyridin-2,4-dicarbonsäure-di-(4-nitrophenyl)ester werden in 25 ml trockenem Dimethylformamid gelöst und mit 0.69 g Alaninmethylester Hydrochlorid und 1.15 ml Triethylamin versetzt. Man läßt zwei Stunden bei Raumtemperatur nachrühren und über Nacht stehen. Die Reaktionsmischung wird in Diethylether aufgenommen und fünf mal mit Wasser gewaschen. Die organische Phase wird mit Natriumsulfat getrocknet und vom Lösungsmittel befreit. Der Rückstand wird über Kieselgel mit Essigester als Laufmittel chromatographiert. Der ölige Rückstand wird mit Pentan/Ether auskristallisiert.
   Schmelzpunkt 96°C; Ausbeute 80 mg
2. Pyridin-2,4-dicarbonsäure-bis(1-benzyloxycarbonyl-2-phenyl-ethyl)amid 2.5 g Pyridin-2,4-dicarbonsäure-di-(4-nitrophenyl)ester werden in 70 ml trockenem Dimethylformamid gelöst und mit 3.56 g Phenylalaninbenzylester Hydrochlorid und 7.0 ml Triethylamin versetzt. Man läßt drei Stunden bei Raumtemperatur nachrühren und über Nacht stehen. Die Reaktionsmischung wird in Diethylether aufgenommen und fünf mal mit Wasser gewaschen. Das Produkt kristallisiert beim Ausschütten aus und wird abgesaugt.
   Schmelzpunkt 104°C; Ausbeute 3.46 g
3. Pyridin-2,4-dicarbonsäure-bis(1-benzyloxycarbonyl-3-methyl-butyl)-amid
   1.02 g Pyridin-2,4-dicarbonsäure-di-(4-nitrophenyl)ester werden in 50 ml trockenem Dimethylformamid gelöst und mit 2.9 g Leucinbenzylester Tosylat und 2 ml Triethylamin versetzt. Man läßt drei Stunden bei Raumtemperatur nachrühren und über Nacht stehen. Die Reaktionsmischung wird in Diethylether aufgenommen und fünf mal mit Wasser gewaschen. Die organische Phase wird mit Natriumsulfat getrocknet und vom Lösungsmittel befreit. Der Rückstand wird über Kieselgel mit Essigester als Laufmittel chromatographiert. Der ölige Rückstand wird mit Pentan/Ether auskristallisiert.
   Schmelzpunkt 82°C; Ausbeute 1.14 mg
4. Pyridin-2,4-dicarbonsäure-bis(1-benzyloxycarbonylethyl) amid
   0.87 g Pyridin-2,4-dicarbonsäure-di-(4-nitrophenyl)ester werden in 30 ml trockenem Dimethylformamid gelöst und mit 1.5 g Alaninbenzylester Tosylat und 1 ml Triethylamin versetzt. Man läßt zwei Stunden bei Raumtemperatur nachrühren und über Nacht stehen. Die Reaktionsmischung wird in Diethylether aufgenommen und fünf mal mit Wasser gewaschen. Die organische Phase wird mit Natriumsulfat getrocknet und vom Lösungsmittel befreit. Der Rückstand wird über Kieselgel mit Toluol/Essigester im Verhältnis 4:1 als Laufmittel chromatographiert. Der ölige Rückstand wird mit Ether verrührt und abgesaugt.
   Schmelzpunkt 103°C; Ausbeute 0.5 g
5. Pyridin-2,4-dicarbonsäure-bis(1-benzyloxycarbonyl-2-(3-indolyl)-ethyl)-amid
   1.02 g Pyridin-2,4-dicarbonsäure-di-(4-nitrophenyl)ester werden in 30 ml trockenem Dimethylformamid gelöst und mit 1.4 g Tryptophanbenzylester und 0.45 ml Triethylamin versetzt. Man läßt drei Stunden bei Raumtemperatur nachrühren und über Nacht stehen. Die Reaktionsmischung wird über Kieselgel mit einer 4:1 Mischung aus Toluol und Essigester als Laufmittel chromatographiert. Der Rückstand wird mit Diisopropylether verrührt und abgesaugt.
   Schmelzpunkt 81°C; Ausbeute 0.9 g
6. Pyridin-2,4-dicarbonsäure-bis(1-methoxycarbonyl-3-methyl-butyl)-amid
   1.5 g Pyridin-2,4-dicarbonsäure-bis-(4-nitrophenyl)ester werden analog Beispiel 1 mit 1.3 g Leucinmethylester Hydrochlorid umgesetzt. Die Reaktionsmischung wird wie in Beispiel 1 beschrieben aufgearbeitet und über Kieselgel mit einer 4:1 Mischung von Toluol/Essigester chromatographiert. Nach Abziehen den Lösungsmittels wird der Rückstand mit Petrolether verrührt und abgesaugt.
   Schmelzpunkt 94°C; Ausbeute 1,0 g
7. Pyridin-2,5-dicarbonsäure-bis(1-benzyloxycarbonyl-3-methyl-propyl)amid
   1.02 g Pyridin-2,5-dicarbonsäure-bis-(4-nitrophenyl)ester werden analog Beispiel 1 mit 1.97 g L-Leucinbenzylester Toluol-4-sulfonat umgesetzt und aufgearbeitet. Das Produkt wird über Kieselgel mit einer 2:1 Mischung von Toluol und Essigester chromatographiert. Nach Abziehen des Lösungsmittels wird das Produkt mit Diisopropylether verrührt und abgesaugt.
   Schmelzpunkt 76°C; Ausbeute 0.38 g
8. Pyridin-2,5-dicarbonsäure-bis(1-benzyloxycarbonyl-2-phenyl-ethyl)-amid
   1.02 g Pyridin-2,5-dicarbonsäure-bis-(4-nitrophenyl)ester werden analog Beispiel 1 mit 1.5 g Phenylalaninbenzylester-hydrochlorid umgesetzt und aufgearbeitet. Das Produkt wird über Kieselgel mit einer 4:1 Mischung von Toluol und Essigester chromatographiert. Nach Abziehen des Lösungsmittels wird das Produkt mit Diethylether verrührt, abgesaugt und aus wenig Essigester unkristallisiert.
   Schmelzpunkt 142°C; Ausbeute 0.9 g
9. Pyridin-2,5-dicarbonsäure-bis(1-benzyloxycarbonyl-2-(3-indolyl)-ethyl)-amid
   1.02 g Pyridin-2,5-dicarbonsäure-bis-(4-nitrophenyl)ester werden analog Beispiel 1 mit 1.4 g Tryptophanbenzylester umgesetzt. Die Reaktionsmischung zur Aufarbeitung in Diethylether aufgenommen und mehrfach mit Wasser gewaschen. Die organische Phase wird getrocknet, vom Lösungsmittel befreit und einmal über Kieselgel mit einer 1,5:1 Mischung von Toluol und Essigester und anschließend noch einmal über Kieselgel mit einer 1:1 Mischung von Cyclohexan und Essigester chromatographiert.
   Schmelzpunkt 92°C; Ausbeute 0.3 g

## Patentansprüche (Patentansprüche für folgende(n) Vertragsstaat(en): AT, BE, CH, DE, FR, GB, IT, LI, LU, NL, SE)

1. Pyridin-2,4- und -2,5-dicarbonsäure-Derivate der Formel I 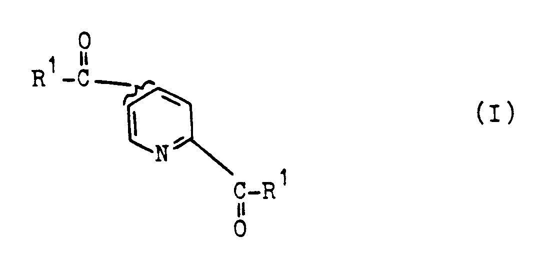 worin
R¹ über N-Terminus gebundene α-Aminosäure oder α-Aminosäurealkylester oder α-Aminosäureamid oder α-Aminosäurealkyl- oder -dialkylamid,
wobei die genannten Alkylreste 1 bis 4 C-Atome aufweisen und gegebenenfalls mit Phenyl monosubstituiert sind und wobei die C₃- und C₄-Alkylreste auch verzweigt sein können
oder
R¹ über N-Terminus gebundenes Di- oder Tripeptid
bedeutet
sowie deren physiologisch verträgliche Salze.

2. Pyridin-2,4- oder -2,5-dicarbonsäure-Derivate der Formel I gemäß Anspruch 1,
worin
R¹ über N-Terminus gebundene α-Aminosäure oder α-Aminosäurealkylester,
wobei der Alkylrest 1 bis 3 C-Atome aufweist und gegebenenfalls mit Phenyl monosubstituiert ist und wobei der C₃-Alkylrest auch verzweigt sein kann
bedeutet
sowie deren physiologisch verträgliche Salze.

3. Verfahren zur Herstellung von Pyridin-2,4- oder -2,5-dicarbonsäure-Derivaten der Formel I gemäß Anspruch 1,
dadurch gekennzeichnet, daß man
eine Verbindung der Formel II, 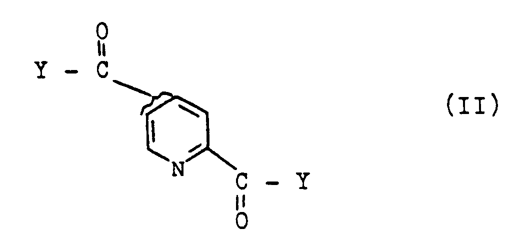 mit einer Verbindung der Formel III
R¹ - H (III)
umsetzt,
wobei R¹ die in Anspruch 1 zu Formel I angegebenen Bedeutungen hat und Y Halogen oder Hydroxy ist oder zusammen mit der Carbonylgruppe einen Aktivester oder ein Anhydrid bildet
und wobei für den Fall, daß R¹ ein über N-Terminus gebundenes Di- oder Tripeptid oder eine über N-Terminus gebundene α-Aminosäure ist, die vorhandene(n) freie(n) Carboxyfunktion(en) gegebenenfalls geschützt ist (sind) und wobei man diese gegebenenfalls verhandene(n) Schutzgruppe(n) nach der Umsetzung hydrolytisch oder hydrogenolytisch unter Bildung der freien Carboxylfunktion(en) abspaltet
und die Reaktionsprodukte gegebenenfalls in ihre physiologisch verträglichen Salze überführt.

4. Verfahren nach Anspruch 3, dadurch gekennzeichnet, daß die Umsetzung unter gleichzeitiger Hinzugabe von Dialkylcarbodiimid erfolgt, wobei die Dialkylreste 1 bis 8 C-Atome aufweisen, die im Falle der C₃-C₈-Verbindungen auch verzweigt oder cyclisch sein können.

5. Verbindungen nach Anspruch 1 oder 2 zur Inhibierung der Prolin- und Lysinhydroxylase.

6. Verbindungen nach Anspruch 1 oder 2 zur Anwendung als Fibrosuppressiva und Immunsuppressiva.

7. Arzneimittel, bestehend aus einer Verbindung gemäß den Ansprüchen 1 oder 2

8. Arzneimittel, enthaltend eine Verbindung gemäß den Ansprüchen 1 oder 2 mit verträglichen pharmazeutischen Trägern.

9. Verfahren zur Herstellung von Arzneimitteln zur Beeinflussung des Stoffwechsels von Collagen und collagenähnlichen Stoffen bzw. der Biosynthese von C1_{q}, dadurch gekennzeichnet, daß man in das Arzneimittel eine Verbindung gemäß den Ansprüchen 1 oder 2 einverleibt.

## Patentansprüche (Patentansprüche für folgende(n) Vertragsstaat(en): ES, GR)

1. Verfahren zur Herstellung von Pyridin-2,4- und -2,5-dicarbonsäure-Derivaten der Formel I 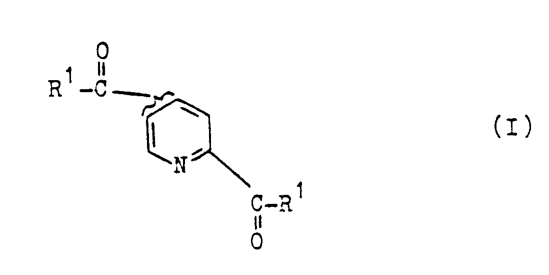 worin
R¹ über N-Terminus gebundene α-Aminosäure oder α-Aminosäurealkylester oder α-Aminosäureamid oder α-Aminosäurealkyl- oder -dialkylamid,
wobei die genannten Alkylreste 1 bis 4 C-Atome aufweisen und gegebenenfalls mit Phenyl monosubstituiert sind und wobei die C₃- und C₄-Alkylreste auch verzweigt sein können
oder
R¹ über N-Terminus gebundenes Di- oder Tripeptid
bedeutet
sowie deren physiologisch verträglichen Salzen, dadurch gekennzeichnet, daß man eine Verbindung der Formel II 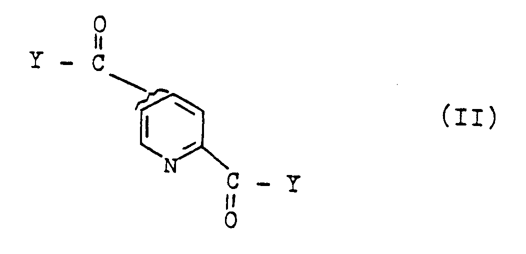 mit einer Verbindung der Formel III
R¹ - H (III)
umsetzt,
wobei R¹ die in Anspruch 1 zu Formel I angegebenen Bedeutungen hat und Y Halogen oder Hydroxy ist oder zusammen mit der Carbonylgruppe einen Aktivester oder ein Anhydrid bildet
und wobei für den Fall, daß R¹ ein über N-Terminus gebundenes Di- oder Tripeptid oder eine über N-Terminus gebundene α-Aminosäure ist, die vorhandene(n) freie(n) Carboxyfunktion(en) gegebenenfalls geschützt ist (sind) und wobei man diese gegebenenfalls verhandene(n) Schutzgruppe(n) nach der Umsetzung hydrolytisch oder hydrogenolytisch unter Bildung der freien Carboxylfunktion(en) abspaltet
und die Reaktionsprodukte gegebenenfalls in ihre physiologisch verträglichen Salze überführt.

2. Verfahren nach Anspruch 1 zur Herstellung von Pyridin-2,4- oder -2,5-dicarbonsäure-Derivaten der Formel I gemäß Anspruch 1,
worin
R¹ über N-Terminus gebundene α-Aminosäure oder α-Aminosäurealkylester,
wobei der Alkylrest 1 bis 3 C-Atome aufweist und gegebenenfalls mit Phenyl monosubstituiert ist und wobei der C₃-Alkylrest auch verzweigt sein kann
bedeutet
sowie deren physiologisch verträglichen Salzen.

3. Verfahren nach Anspruch 1 oder 2, dadurch gekennzeichnet, daß die Umsetzung unter gleichzeitiger Hinzugabe von Dialkylcarbodiimid erfolgt, wobei die Dialkylreste 1 bis 8 C-Atome aufweisen, die im Falle der C₃-C₈-Verbindungen auch verzweigt oder cyclisch sein können.

4. Verfahren zur Herstellung von Arzneimitteln zur Inhibierung der Prolin- und Lysinhydroxylase, dadurch gekennzeichnet, daß man eine Verbindung gemäß den Ansprüchen 1, 2 oder 3 in eine geeignete Darreichungsform bringt.

5. Verfahren zur Herstellung von Arzneimitteln zur Anwendung als Fibrosuppressiva und Immunsuppressiva, dadurch gekennzeichnet, daß man eine Verbindung gemäß den Ansprüchen 1,2 oder 3 in eine geeignete Darreichungsform bringt.

6. Verfahren zur Herstellung von Arzneimitteln zur Beeinflussung des Stoffwechsels von Collagen und collagen-ähnlichen Stoffen bzw. der Biosynthese von C1_{q}, dadurch gekennzeichnet, daß man eine Verbindung gemäß den Ansprüchen 1, 2 oder 3 in eine geeignete Darreichungsform bringt.

## Claims (Claims for the following Contracting State(s): AT, BE, CH, DE, FR, GB, IT, LI, LU, NL, SE)

1. Pyridine-2,4- and -2,5-dicarboxylic acid derivatives of the formula I 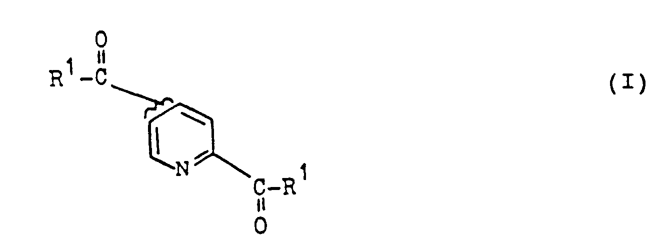 in which
R¹ denotes an α-amino acid or α-amino acid alkyl ester or α-amino acid amide or α-amino acid alkyl-or dialkylamide which is bonded via the N terminus and in which the said alkyl radicals have 1 to 4 carbon atoms and are optionally monosubstituted by phenyl, and in which the C₃- and C₄-alkyl radicals can also be branched,
or
R¹ denotes a di- or tripeptide which is bonded via the N terminus,
and their physiologically tolerated salts.

2. Pyridine-2,4- or -2,5-dicarboxylic acid derivatives of the formula I as claimed in claim 1,
in which
R¹ denotes an α-amino acid or α-amino acid alkyl ester which is bonded via the N terminus and in which the alkyl radical has 1 to 3 carbon atoms an is optionally monosubstituted by phenyl an in which the C₃-alkyl radical can also be branched,
and their physiologically tolerated salts.

3. A process for the preparation of pyridine-2,4- or -2,5-dicarboxylic acid derivatives of the formula I as claimed in claim 1, which comprises reaction of a compound of the formula II 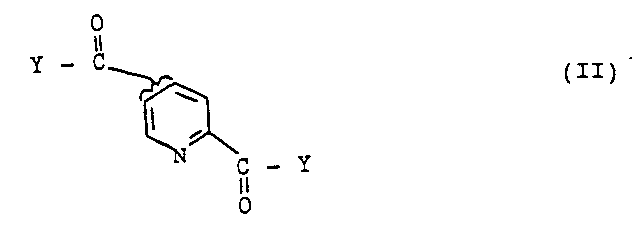 with a compound of the formula III
R¹ - H (III)
in which
R¹ has the meanings indicated for formula I in claim 1, and Y is halogen or hydroxyl or, together with the carbonyl group, forms an active ester or an anhydride, and in which, in the case where R¹ is a di- or tripeptide which is bonded via the N terminus or an α-amino acid which is bonded via the N terminus, the free carboxyl group(s) which is (are) present is (are) optionally protected, and in which this (these) protective group(s) which is (are) optionally present is (are) eliminated after the reaction by hydrolysis or hydrogenolysis to form the free carboxyl group(s), and conversion of the reaction products, where appropriate, into their physiologically tolerated salts.

4. The process as claimed in claim 3, wherein the reaction is carried out with simultaneous addition of dialkylcarbodiimide in which the dialkyl radicals have 1 to 8 carbon atoms and which, in the case of the C₃-C₈ compounds, can also be branched or cyclic.

5. A compound as claimed in claim 1 or 2 for inhibiting proline hydroxylase and lysine hydroxylase.

6. A compound as claimed in claim 1 or 2 for use as fibrosuppressant and immunosuppressant.

7. A medicament composed of a compound as claimed in claim 1 or 2.

8. A medicament containing a compound as claimed in claim 1 or 2 with tolerated pharmaceutical vehicles.

9. A process for the preparation of medicaments for influencing the metabolism of collagen and collagenlike substances and the biosynthesis of C1q, which comprises incorporating a compound as claimed in claim 1 or 2 into the medicament.

## Claims (Claims for the following Contracting State(s): ES, GR)

1. A process for the preparation of pyridine-2,4- and -2,5-dicarboxylic acid derivatives of the formula I 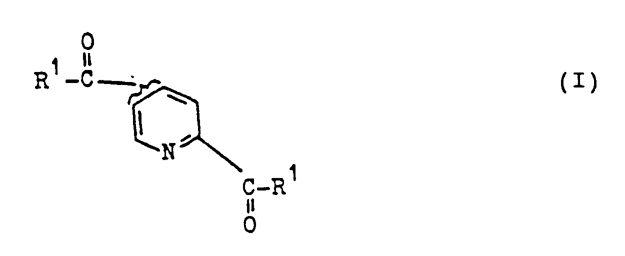 in which
R¹ denotes an α-amino acid or α-amino acid alkyl ester or α-amino acid amide or α-amino acid alkyl-or dialkylamide which is bonded via the N terminus and in which the said alkyl radicals have 1 to 4 carbon atoms and are optionally monosubstituted by phenyl, and in which the C₃- and C₄-alkyl radicals can also be branched,
or
R¹ denotes a di- or tripeptide which is bonded via the N terminus,
and their physiologically tolerated salts,
which comprises reaction of a compound of the formula II 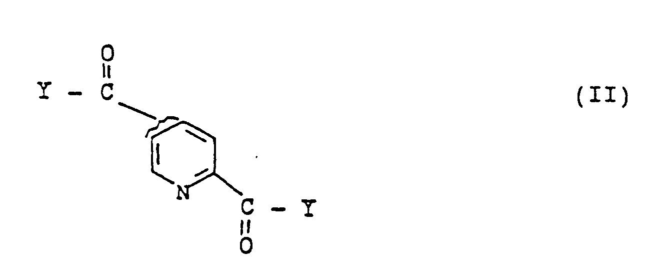 with a compound of the formula III
R¹ - H (III)
in which
R¹ has the meanings indicated for formula I in claim 1, and Y is halogen or hydroxyl or, together with the carbonyl group, forms an active ester or an anhydride, and in which, in the case where R¹ is a di- or tripeptide which is bonded via the N terminus or an α-amino acid which is bonded via the N terminus, the free carboxyl group(s) which is (are) present is (are) optionally protected, and in which this (these) protective group(s) which is (are) optionally present is (are) eliminated after the reaction by hydrolysis or hydrogenolysis to form the free carboxyl group(s), and conversion of the reaction products, where appropriate, into their physiologically tolerated salts.

2. The process as claimed in claim 1 for the preparation of pyridine-2,4- or -2,5-dicarboxylic acid derivatives of the formula I as claimed in claim 1,
in which
R¹ denotes an a-amino acid or α-amino acid alkyl ester which is bonded via the N terminus and in which the alkyl radical has 1 to 3 carbon atoms and is optionally monosubstituted by phenyl and in which the C₃-alkyl radical can also be branched,
and their physiologically tolerated salts.

3. The process as claimed in claim 1 or 2, wherein the reaction is carried out with simultaneous addition of dialkylcarbodiimide in which the dialkyl radicals have 1 to 8 carbon atoms and which, in the case of the C₃-C₈ compounds, can also be branched or cyclic.

4. A process for the preparation of medicaments for the inhibition of proline hydroxylase and lysine hydroxylase, which comprises converting a compound as stated in claim 1, 2 or 3 into a form suitable for administration.

5. A process for the preparation of medicaments for use as fibrosuppressants and immunosuppressants, which comprises converting a compound as stated in claim 1, 2 or 3 into a form suitable for administration.

6. A process for the preparation of medicaments for influencing the metabolism of collagen and collagenlike substances and the biosynthesis of C1q, which comprises converting a compound as stated in claim 1, 2 or 3 into a form suitable for administration.

## Revendications (Revendications pour l'(les) Etat(s) contractant(s) suivant(s): AT, BE, CH, DE, FR, GB, IT, LI, LU, NL, SE)

1. Dérivés des acides pyridine-2,4- et pyridine-2,5-dicarboxyliques, de formule I: 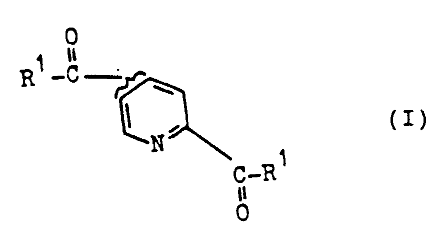 où
R¹ représente un α-aminoacide lié par sa terminaison N ou un ester alkylique d'α-aminoacide ou un amide d'α-aminoacide
ou un alkylamide ou dialkylamide d'α-aminoacide,
les radicaux alkyle cités présentant 1 à 4 atomes de carbone et étant éventuellement monosubstitués par un radical phényle, et les radicaux alkyle en C₃ et C₄ pouvant également être ramifiés,
ou
R¹ représente un dipeptide ou tripeptide lié par sa terminaison N,
ainsi que leurs sels physiologiquement acceptables.

2. Dérivés des acides pyridine-2,4- ou pyridine-2,5-dicarboxyliques, de formule I, selon la revendication 1,
où
R¹ représente un α-aminoacide lié par sa terminaison N ou un ester alkylique d'α-aminoacide,
le radical alkyle présentant 1 à 3 atomes de carbone et étant éventuellement monosubstitué par un radical phényle, et le radical alkyle en C₃ pouvant également être ramifié,
ainsi que leurs sels physiologiquement acceptables.

3. Procédé pour préparer des dérivés des acides pyridine-2,4- ou pyridine-2,5-dicarboxyliques, de formule I, selon la revendication 1, caractérisé en ce qu'on fait réagir un composé de formule II, 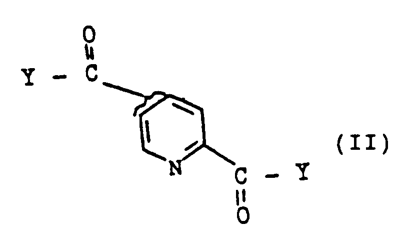 avec un composé de formule III
R¹ - H (III)
R¹ présentant les significations donnée à la formule I dans la revendication 1 et Y étant un atome d'halogène ou un groupe hydroxy, ou formant avec le groupe carbonyle un ester actif ou un anhydride,
et, dans le cas où R¹ est un dipeptide ou tripeptide lié par sa terminaison N ou un α-aminoacide lié par sa terminaison N, la(les) fonction(s) carboxy libre(s) présente(s) étant éventuellement protégée(s),
et où, après la réaction, on élimine ce(s) groupe(s) protecteur(s) éventuellement présent(s), par voie d'hydrolyse ou d'hydrogénolyse, en formant la(les) fonction(s) carboxy libre(s),
et on transforme les produits de réaction, le cas échéant, en leurs sels physiologiquement acceptables.

4. Procédé selon la revendication 3, caractérisé en ce qu'on effectue la réaction avec addition simultanée de carbodiimide dialkylique, les radicaux alkyle présentant 1 à 8 atomes de carbone et, dans le cas de composés en C₃ à C₈, pouvant également être ramifiés ou cycliques.

5. Composés selon la revendication 1 ou 2, pour inhiber l'hydroxylase de la proline et l'hydroxylase de la lysine.

6. Composés selon la revendication 1 ou 2, à utiliser en tant qu'agents fibro-suppresseurs et agents immuno-suppresseurs.

7. Médicament constitué d'un composé selon les revendications 1 ou 2.

8. Médicament contenant un composé selon les revendications 1 ou 2, avec des véhicules pharmaceutiques acceptables.

9. Procédé pour fabriquer des médicaments permettant d'influer sur le métabolisme du collagène et des substances semblables au collagène notamment sur la biosynthèse du C1_{q}, caractérisé en ce qu'on incorpore dans le médicament un composé selon les revendications 1 ou 2.

## Revendications (Revendications pour l'(les) Etat(s) contractant(s) suivant(s): ES, GR)

1. Procédé pour préparer des dérivés des acides pyridine-2,4- et pyridine-2,5-dicarboxyliques, de formule I: 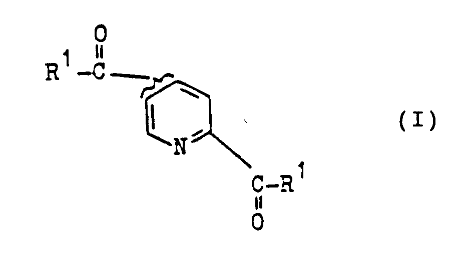 où
R¹ représente un α-aminoacide lié par sa terminaison N ou un ester alkylique d'α-aminoacide ou un amide d'α-aminoacide
ou un alkylamide ou dialkylamide d'α-aminoacide, les radicaux alkyle cités présentant 1 à 4 atomes de carbone et étant éventuellement monosubstitués par un radical phényle, et les radicaux alkyle en C₃ et C₄ pouvant également être ramifiés,
ou
R¹ représente un dipeptide ou tripeptide lié par sa terminaison N,
ainsi que leurs sels physiologiquement acceptables,
caractérisé en ce qu'on fait réagir un composé de formule II, 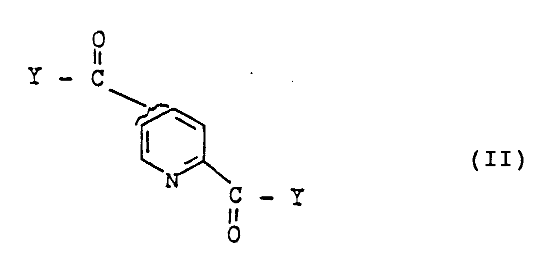 avec un composé de formule III
R¹ - H (III)
R¹ présentant la signification donnée à la formule I dans la revendication 1 et Y étant un atome d'halogène ou un groupe hydroxy, ou formant avec le groupe carbonyle un ester actif ou un anhydride,
et, dans le cas où R¹ est un dipeptide ou tripeptide lié par sa terminaison N ou un α-aminoacide lié par sa terminaison N, la(les) fonction(s) carboxy libre(s) présente(s) étant éventuellement protégée(s),
et où, après la réaction, on élimine ce(s) groupe(s) protecteur(s) éventuellement présent(s), par voie d'hydrolyse ou d'hydrogénolyse, en formant la(les) fonction(s) carboxy libre(s),
et on transforme les produits de réaction, le cas échéant, en leurs sels physiologiquement acceptables.

2. Procédé selon la revendication 1 pour préparer des dérivés des acides pyridine-2,4- ou pyridine-2,5-dicarboxyliques, de formule I, selon la revendication 1,
où
R¹ représente un α-aminoacide lié par sa terminaison N ou un ester alkylique d'α-aminoacide,
le radical alkyle présentant 1 à 3 atomes de carbone et étant éventuellement monosubstitué par un radical phényle, et le radical alkyle en C₃ pouvant également être ramifié,
ainsi que leurs sels physiologiquement acceptables.

3. Procédé selon la revendication 1 ou 2, caractérisé en ce qu'on effectue la réaction avec addition simultanée de carbodiimide dialkylique, les radicaux alkyle présentant 1 à 8 atomes de carbone et, dans le cas de composés en C₃ à C₈, pouvant également être ramifiés ou cycliques.

4. Procédé pour fabriquer des médicaments destinés à inhiber l'hydroxylase de la proline et l'hydroxylase de la lysine, caractérisé en ce qu'on met un composé selon les revendications 1, 2 ou 3, sous une forme galénique appropriée.

5. Procédé pour fabriquer des médicaments à utiliser en tant qu'agents fibro-suppresseurs et agents immuno-suppresseurs, caractérisé en ce qu'on met un composé selon les revendications 1, 2 ou 3, sous une forme galénique appropriée.

6. Procédé pour fabriquer des médicaments permettant d'influer sur le métabolisme du collagène et des substances semblables au collagène notamment sur la biosynthèse du C1_{q}, caractérisé en ce qu'on met un composé selon les revendications 1, 2 ou 3, sous une forme galénique appropriée.
